# EUROPEAN PATENT SPECIFICATION

(11) **EP 2 279 729 B1**
(45) Date of publication and mention of the grant of the patent: **17.08.2016**
(21) Application number: 10075739.2
(22) Date of filing: 14.07.2004
(51) Int. Cl.: A61K 9/48

(54) **Controlled release preparations**
Zubereitungen mit kontrollierter Freisetzung
Préparations à libération controllée

(30) Priority: 17.07.2003 US 487968 P
(43) Date of publication of application: 02.02.2011
(62) Divisional of application: 04778128.1
(73) Proprietor: Banner Life Sciences, LLC, High Point, North Carolina 27265 (US)
(72) Inventor: Hassan, EmadEldin M., High Point North Carolina 27265 (US); Chidambaram, Nachiappan, Salt Lake City, Utah 84105 (US); Fatmi, Aqeel A., Greensboro North Carolina 27410 (US)
(74) Representative: Potter Clarkson LLP

(56) References cited:
- EP-A2- 0 212 875
- GB-A- 1 015 251
- US-A- 4 695 450

## Description

### Field of the Invention

This invention relates generally to controlled release preparations and soft capsules. The invention relates further to emulsions and suspensions, including compositions and methods of manufacturing controlled release capsules where the fill contains a suspension and/or an emulsion.

### Background of the Invention

Controlled release preparations have been a vital development in healthcare sciences. One advantage of such medicaments is improved patient compliance, especially where patients are under multiple or chronic treatments. Regarding the need to increase compliance rates, it is noted that the growing population of elder people further increases the demand for controlled release medication. Elderly patients often have particular difficulty with compliance for multiple daily dosages, especially in the context of a multiplicity of required medications.

While patient compliance is an immediate benefit of controlled release products, minimization of side effects of potent medicines is also a desirable advantage of controlled release preparations. For example, tachycardia, a well-known side effect of the cardiovascular drug nifedipine, can be significantly controlled when the drug is administered in a controlled release form. In fact, using controlled release preparations helps avoid sudden high drug concentrations of drugs in the systemic circulation and reduces subsequent adverse effects or toxicity.

Oral controlled release technologies are classified generally as of "matrix" or "film" nature. The matrix type is mainly used in tablets using polymeric or lipid materials that control both penetration of water and the release of the active ingredient to the surrounding environment. For example, U.S. Pat. No 4,882,167 describes tablet compositions containing a hydrophobic carbohydrate polymer, *e.g.* ethyl cellulose and a wax material such as carnauba wax and made by direct compression. Despite of the apparent simplicity of the direct compression technique, it has limitations when applied to low dose, potent active ingredients. The low amounts of potent drugs are hardly well distributed in a directly compressed matrix due to the uncontrolled differences in particle size and density between the drug and matrix particles. Such differences usually lead to lack of homogeneous distribution of the drug in the matrix and lack of content uniformity. To overcome the limitations of direct compression matrix manufacture, a wet granulation technique is often applied. An example of the wet granulation procedure is described in U.S. Pat. No. 6,572,889 to Guo where granulation of active materials such as cabamazepine is performed in presence of water and polymeric substances. While wet granulation basically improves the distribution of an active material in a matrix, it is still considered a tedious and time-consuming technique.

The second major technology for oral controlled release preparations is applying coating or films to control the drug release from particles (e.g., pellets or microcapsules) or unit doses such as tablets. U.S. Pat Nos. 5,871,776 and 4,572,833 provide details of preparing controlled release particles that can be filled into hard gelatin capsules or compressed into tablets. While pellets or microcapsules are fairly popular in controlled release products, they are considered an intermediate product that requires additional manufacturing steps to produce as a useful dosage form. suitable for direct consumption by patients. On the other hand, coating unit doses such as tablets seems to be a more direct approach to manufacture oral controlled release pharmaceuticals. Tablet coating for controlled release purposes has been quite well known in the pharmaceutical industry for a long period of time and is well illustrated in standard pharmaceutical text books (see for example Remington's Pharmaceutical Industries, 18th edition, Pages 1666 to 1675. Alfonso Gennaro, editor, Mack Publishing Co. Easto, PA, 1990). As experienced persons in the art would expect, unit dose coating has many drawbacks that may lead to performance failures due to defects in the coat, such as pinholes and sticking.

Soft capsules have been tested as a controlled drug delivery system by Cohen, et al. (U.S. Pat. No. 4,795,642), where an aqueous fill of the polysaccharide gum sodium alginate forms a gel in presence of cationic elements such as heavy metal ions. However, the manufacture of soft capsules is presently the least utilized technique for producing oral controlled release preparations.

US4695450 discloses anhydrous emulsions comprising active agents which can be encapsulated, particularly in soft gelatin capsules. The emulsions comprise an oil phase (e.g. vegetable oil) and a phase of water-soluble, but anhydrous liquids, e.g. polyethylene glycols with a molecular weight of 300-20000. The hydrophilic phase can further comprise cellulose esters or ethers as swelling or thickening agents.

### Summary of the Invention

The present invention provides numerous matrix systems based on lipids and lipophilic materials either alone or in presence of a hydrophilic phase. The described matrices have a hydrophobic surface in contact with the hydrophilic capsule shell to minimize any potential shell-fill interactions, as described elsewhere when soft capsules are filled with hydrophilic materials such as polyethylene glycol or similar vehicles.

This invention provides a controlled release soft capsule having a shell and a matrix fill comprising an active ingredient or drug, wherein the matrix fill consists of two phases in the form of an emulsion, wherein the emulsion comprises a hydrophilic internal phase consisting essentially of polyethylene glycol of molecular weight ranging from about 200 to about 8000, methyl cellulose and an active ingredient or drug and a lipid or lipophilic external phase; and wherein the ratio of the internal phase to external phase is from about 0.5:10 to about 1:1 by weight and a method of manufacturing a controlled release soft capsule, the method comprising
a) dispersing the active ingredient or drug in an internal phase to form a clear solution or suspension using a propeller or homogenizer mixer;
b) adding the internal phase to a molten external phase containing at least one surfactant in an amount from about 0.1 % to about 5% by weight to form a resulting mixture;
c) forming an emulsion from the resulting mixture by subjecting the mixture to mechanical forces generated by a propeller mixer, a homogenizer, or a microfluidizer;
d) cooling the emulsion to from about 20°C to about 35°C; and
e) encapsulating the emulsion using a rotary die encapsulation machine to form the controlled release capsule.

The present invention also provides compositions and methods of manufacture of controlled release medicaments in the soft gel dosage form. The invention also provides methods for manufacture of the fill of a controlled release soft gel in the form of a suspension, where part or all of the active ingredient or drug is suspended or dissolved in a two-phase matrix. Also provided are compositions and methods where the active ingredient or drug of a medicament is incorporated in a two-phase matrix. The two-phase matrix comprises an emulsion of hydrophilic material as the internal phase, and a hydrophobic a lipid or lipophilic external phase external phase.

In one aspect, the invention relates to a controlled release soft capsule having a shell and a matrix fill, wherein the matrix fill includes an active ingredient or drug incorporated as solid particles in lipid or lipophilic materials. The lipid or lipophilic material can be a vegetable oil, hydrogenated vegetable oil, fatty acid, wax, fatty acid ester, or a combination thereof. The matrix fill can include a release regulator which can be a fatty acid salt, fatty acid ester, or fatty acid polyoxyethylene derivative. The release regulator can be a surfactant having an hydrophilic/lipophilic balance (HLB) value between about 3 and about 40.

The active ingredient or drug can be a non-steroid anti-inflammatory drug or an anti-asthmatic. The active ingredient or drug can be diclofenac, naproxene, ibuprofen, ketoprofen, celecoxib, or theophylline. The ratio of the active ingredient or drug to the matrix fill can be from about 1:9 to about 1:1 by weight. The ratio can also be from about 1:8 to about 1:1 by weight.

The present invention relates to a controlled release soft capsule having a shell and a matrix fill including an active ingredient or drug, wherein the physical state of the matrix can be a semi-fluid, or a structured solid state. In some embodiments, the matrix can be a fluid or semi-fluid at room temperature, or at a body temperature of a subject to which the capsule is intended to be administered. In some embodiments, the active ingredient or drug can be partially soluble in the matrix and at least a portion of the active ingredient or drug can be in solid form in the matrix.

The present invention relates to a controlled release soft capsule including a shell and a matrix fill, wherein the matrix fill includes two phases in the form of an emulsion. In some embodiments, the emulsion can be a water-in-oil type emulsion. The emulsion can include a surfactant or combination of surfactants having HLB values ranging from about 2 to about 20. The HLB values can also range from about 5 to about 15.

In some embodiments, the active ingredient or drug can be an anti-asthmatic, narcotic analgesic, narcotic antagonist, or cardiovascular drug. The active ingredient or drug can be diltiazem, nifedipine, oxycodone, morphine, morphine analogues, or morphine antagonists.

In some embodiments, the ratio of the active ingredient or drug to the matrix fill can be from about 1:100 to about 1:2 by weight. The ratio can also be from about 1:50 to about 1:3 by weight.

The emulsion includes a hydrophilic internal phase and a lipid or lipophilic external phase. The internal phase consists essentially of polyethylene glycol of molecular weight ranging from about 200 to about 8000, methyl cellulose and an active ingredient or drug. The internal phase can also be structured.

The external phase can include a vegetable oil, hydrogenated vegetable oil, fatty acid, wax, fatty acid ester, or a combination thereof.

The active ingredient or drug can be dispersed in the internal phase as a solution or suspension form.

The ratio of the internal phase to external phase is from about 0.5:10 to about 1:1 by weight. The ratio can also be from about 1:9 to about 1:1 by weight.

In another aspect, the invention relates to a controlled release soft capsule having a shell and a matrix fill, wherein the matrix fill includes two phases in the form of an emulsion, with an active ingredient or drug distributed in both an external and internal phase. The active ingredient or drug can be in the form of solid particles. The active ingredient or drug can be present as solid particles incorporated in both the internal phase and the external phase.

Also described herein is a method of manufacturing a matrix fill for a controlled release soft capsule including applying heat to the matrix components during mixing or prior to mixing at about the melting point of the matrix fill composition; and mixing the active ingredient or drug with the lipid or lipophilic matrix ingredients using mechanical or ultrasonic forces to form the matrix fill. The matrix fill can be flowable such that it can be encapsulated using a rotary die encapsulation machine. The matrix components can be heated to a temperature in the range of from about 25°C to about 70°C. The matrix components can also be heated to a temperature in the range of from about 30°C to about 50°C.

The present invention relates to a method of manufacturing a controlled release soft capsule, wherein the matrix fill includes two phases in the form of an emulsion. The method includes (a) dispersing the active ingredient or drug in an internal phase to form a clear solution or suspension using propeller or homogenizer mixers; (b) adding the internal phase materials to a molten external phase containing at least one surfactant in an amount from about 0.1% to about 5% by weight to form a resulting mixture; (c) forming an emulsion from the resulting mixture by subjecting the mixture to mechanical forces generated by a propeller mixer, a homogenizer, or a microfluidizer; (d) cooling the emulsion to from about 20°C to about 35°C; and (e) encapsulating the emulsion using a rotary die encapsulation machine to form the controlled release capsule.

### Detailed Description of the Invention

The controlled release soft capsules according to the invention comprise a shell and a matrix fill. The matrix fill is an emulsion-type matrix.

As described herein in a suspension-type matrix fill, the active ingredient or drug is incorporated in the matrix fill as solid particles in lipid or lipophilic materials such as vegetable oils, hydrogenated vegetable oils, fatty acids, waxes, or fatty acid esters, or a combination thereof. The matrix composition may further contain a release regulator to modify the release profile to suit an optimum therapeutic requirement. The release regulator can be a surface-active agent that enhances water penetration into the lipid or lipophilic matrix to increase drug release. Examples of release regulators are fatty acid slats, fatty acid esters, or fatty acid polyoxyethylene derivatives. Surfactants having HLB values between about 3 and about 40 can be selected as release regulators. The hydrophilic/lipophilic balance (HLB) characteristic of surfactants can be determined in accordance with "Physical Pharmacy: Physical Chemical Principles in the Pharmaceutical Sciences," Fourth Edition, pp. 371-373, A. Martin, Ed., Lippincott Williams & Wilkins, Philadelphia (1993).

In a suspension-type matrix fill, as described herein, the matrix, at room or body temperature, can be in a fluid or structured solid state (solid, semi-solid, or gel). The drug can be partially soluble in the matrix while the rest of the drug is in a solid form. The presence of drug in two physical forms, solid particles and solution, can be useful by providing dual release patterns where one drug state is released faster than the other form.

The invention relates to emulsion-type fills. Such fills are described herein as "emulsion-type" fills because they comprise an emulsion. The matrix fills for these embodiments can be characterized generally as emulsion-type fills, even though the active ingredient or drug can be present as a suspension in one or more phases of the emulsions of embodiments as described herein.

The soft gel matrix fill of the present invention comprises two phases in the form of an emulsion (emulsion-type matrix). The emulsion can be a water-in-oil type emulsion. The hydrophilic internal phase consists essentially of polyethylene glycol of molecular weight ranging from about 200 to about 8000, methylcellulose, and an active ingredient or drug. The internal phase state can be "fluid" or "structured." A "fluid" internal phase, as used herein, means a completely flowable liquid whose globules can aggregate to make a larger globule. A "structured" internal phase, as used herein, means a solid, semisolid or a gel whose shape is relatively stable and does not usually aggregate to form a large globule. A structured internal phase therefore provides more controlled drug release and stabilizes the physical state of the matrix.

The external phase of the matrix fill emulsion comprises lipid or lipophilic materials similar to those described above. The active ingredient or drug can be dispersed in the internal phase as a solution and/or as a suspension. The emulsion matrix can contain a surfactant or combination of surfactants having HLB values ranging from about 2 to about 20. The HLB range can also be from about 5 to about 15.

Where the matrix fill is of an emulsion type, the drug is distributed in both external and internal phases. One portion of the active ingredient or drug in form of solid particles can be incorporated in the internal phase, while another portion is dispersed in the external phase as solid particles.

This invention also provides methods for making controlled release products in a soft capsule form. The methods are applicable for production of controlled release preparations of low dose (potent) drugs that are highly water-soluble. The methods are also suitable for preparing controlled release products of relatively less potent, moderately water-soluble drugs.

Suspension-type matrix fill compositions can be used for drugs that are moderately water-soluble at a dosage of between about 25 mg to about 500 mg. Such

drugs include non-steroid anti-inflammatory drugs and anti-asthmatics, e.g., diclofenac, naproxene, ibuprofen, ketoprofen, celecoxib, and theophylline.

The emulsion-type matrix fill of the present invention can be used for highly water-soluble molecules such as anti-asthmatics, narcotic analgesics, and analgesic antagonists as well as cardiovascular drugs, *e.g.,* diltiazem, nifedipine, oxycodone, morphine, morphine analogues, and morphine antagonists.

The suspension-type matrix fill can be manufactured by mixing the active ingredient or drug with the lipid or lipophilic matrix ingredients using mechanical or ultrasonic forces. Applying heat while or prior to mixing has the benefit of reducing the matrix viscosity. Reduced matrix viscosity in turn results in more efficient mixing. The matrix materials can be heated to temperature at or close to the melting point of the matrix composite. The melting point of the composite matrix is workable in the range of from about 25°C to about 70°C. The melting point range of the matrix composition can also be from about 30°C to about 50°C. The drug-to-matrix ratio can be concentrated enough to provide a low total mass per unit dose, yet can still be flowable to allow encapsulation using a rotary die encapsulation machine. A workable drug-to-matrix ratio range is from about 1:9 to about 1:1 by weight. The drug-to-matrix ratio range can also be from about 1:8 to about 1:1 by weight.

The emulsion-type of matrix fill of the present invention can be manufactured by dispersing the active ingredient or drug in the internal phase to provide a clear solution or suspension. The active ingredient or drug can be dispersed using propeller or homogenizer mixers. The internal phase materials can then be added to the molten external phase containing surfactant from about 0.1% to about 5% by weight. The emulsion can be made using mechanical forces generated by a propeller mixer, a homogenizer, or a microfluidizer. The matrix is then cooled to a temperature of from about 20°C to about 35°C for encapsulation using a rotary die encapsulation machine. The internal-to-external phase workable ratio is from about 0.5:10 to about 1:1 by weight. The ratio range can also be from about 1:9 to about 1:1 by weight. The workable drug-to-matrix ratio can be from about 1:100 to about 1:2 by weight. The range of the drug-to-matrix can also be from about 1:50 to about 1:3 by weight.

Also described herein are matrix fills that are encapsulated in hard shell capsules. Guidance regarding hard shell, liquid filling technology can be found in Walker, S.E., et al., "The filling of molten and thixotropic formulations into hard gelatin capsules," J. Pharm. Pharmacol. 32:389 - 393 (1980); McTaggert, C., et al., "The evaluation of an automatic system for filling liquids into hard gelatin capsules," J. Pharm. Pharmacol. 36:119 - 121 (1984); Hawley, A.R. et al., "Physical and chemical characterization of thermosoftened bases for molten filled hard gelatin capsule formulations," Drug. Devel. Ind. Pharm. 18(16):1719 (1992); and Cade, D., et al., "Liquid filled and sealed hard gelatin capsules," Acta Pharm. Technol. 33(2):97 - 100 (1987).

The following Examples are intended for purposes of illustration only, and should not be interpreted as limiting in any way of the scope of the invention.

### Examples

**Formulation 1:**

| *Ingredients* | *Amount (% w*/*w)* |
|---|---|
| Diltiazem Hydrochloride | 5.00 |
| Soybean Oil | 6.24 |
| Vegetable Shortening | 60.00 |
| Vegetable Flakes | 12.00 |
| Glyceryl mono oleate | 2.35 |
| Span 60* | 0.16 |
| Methyl Cellulose | 1.50 |
| PEG 3350 | 4.50 |
| PEG 400 | 8.25 |

| | |
|---|---|
| *sorbitan stearate. | |

### Procedure:

Vegetable shortening, vegetable flakes, Glyceryl mono oleate, Span 60 and soybean oil were melted together at 50° to 70°C (wax or lipophilic phase). Methylcellulose, PEG 3350 and PEG 400 were melted separately at 50° to 70°C (aqueous phase). Diltiazem hydrochloride was dispersed in the melted aqueous phase and added slowly to the wax phase with homogenization, while maintaining the temperature between 50° and 70°C. The resultant homogeneous emulsion phase was cooled and encapsulated.

### Evaluation:

Filled capsules were subjected to dissolution as per USP using the paddle method in distilled water at 100 RPM.

### Result:

T₅₀ (time required for 50% dissolution) is about 18h.

Note: The Procedure and Evaluation followed for Formulation 1 was also used for Formulations 2-24 below.

**Formulation 2:**

| *Ingredients* | *Amount (% w*/*w)* |
|---|---|
| Diltiazem Hydrochloride | 5.00 |
| Soybean Oil | 27.84 |
| Vegetable Shortening | 38.40 |
| Vegetable Flakes | 12.00 |
| Glyceryl mono oleate | 2.35 |
| Span 60 | 0.16 |
| Methyl Cellulose | 1.50 |
| PEG 3350 | 4.50 |
| PEG 400 | 8.25 |

**Result:** T₅₀ (time required for 50% dissolution) is about 3h.

**Formulation 3:**

| *Ingredients* | *Amount (%* w/w) |
|---|---|
| Diltiazem Hydrochloride | 5.00 |
| Soybean Oil | 23.84 |
| Vegetable Shortening | 42.40 |
| Vegetable Flakes | 12.00 |
| Glyceryl mono oleate | 2.35 |
| Span 60 | 0.16 |
| Methyl Cellulose | 1.50 |
| PEG 3350 | 4.50 |
| PEG 400 | 8.25 |

**Result:** T₅₀ (time required for 50% dissolution) is about 1h.

**Formulation 4:**

| *Ingredients* | *Amount* (% *w*/*w)* |
|---|---|
| Diltiazem Hydrochloride | 10.00 |
| Soybean Oil | 4.68 |
| Vegetable Shortening | 44.70 |
| Vegetable Flakes | 9.00 |
| Glyceryl mono oleate | 2.70 |
| Span 60 | 0.12 |
| Lecithin | 0.30 |
| Methyl Cellulose | 3.00 |
| PEG 3350 | 9.00 |
| PEG 400 | 16.50 |

**Result:** T₅₀ (time required for 50% dissolution) is about 4h.

**Formulation 5:**

| *Ingredients* | *Amount (%* w/w) |
|---|---|
| Diltiazem Hydrochloride | 10.00 |
| Soybean Oil | 20.88 |
| Vegetable Shortening | 25.50 |
| Vegetable Flakes | 12.00 |
| Glyceryl mono oleate | 2.70 |
| Span 60 | 0.12 |
| Lecithin | 0.30 |
| Methyl Cellulose | 3.00 |
| PEG 3350 | 9.00 |
| PEG 400 | 16.50 |

**Result:** T₅₀ (time required for 50% dissolution) is about 8h.

**Formulation 6:**

| *Ingredients* | *Amount (% w*/*w)* |
|---|---|
| Diltiazem Hydrochloride | 10.00 |
| Soybean Oil | 20.88 |
| Vegetable Shortening | 28.50 |
| Vegetable Flakes | 9.00 |
| Glyceryl mono oleate | 2.70 |
| Span 60 | 0.12 |
| Lecithin | 0.30 |
| Methyl Cellulose | 3.00 |
| PEG 3350 | 12.00 |
| PEG 400 | 13.50 |

**Result:** T₅₀ (time required for 50% dissolution) is about 3.5h.

**Formulation 7:**

| *Ingredients* | *Amount (% w*/*w)* |
|---|---|
| Diltiazem Hydrochloride | 10.00 |
| Soybean Oil | 27.00 |
| Vegetable Shortening | 13.88 |
| Vegetable Flakes | 18.00 |
| Glyceryl mono oleate | 2.70 |
| Span 60 | 0.12 |
| Lecithin | 0.30 |
| Methyl Cellulose | 3.00 |
| PEG 3350 | 9.00 |
| PEG 400 | 16.50 |

**Result:** T₅₀ (time required for 50% dissolution) is about 4h.

**Formulation 8:**

| *Ingredients* | *Amount (% w*/*w)* |
|---|---|
| Diltiazem Hydrochloride | 10.00 |
| Soybean Oil | 27.00 |
| Vegetable Shortening | 13.88 |
| Vegetable Flakes | 18.00 |
| Glyceryl mono oleate | 2.70 |
| Span 60 | 0.12 |
| Lecithin | 0.30 |
| Methyl Cellulose | 3.00 |
| PEG 3350 | 12.00 |
| PEG 400 | 13.50 |

**Result:** T₅₀ (time required for 50% dissolution) is about 11h.

**Formulation 9:**

| *Ingredients* | *Amount (% wlw)* |
|---|---|
| Diltiazem Hydrochloride | 10.00 |
| Soybean Oil | 23.38 |
| Vegetable Shortening | 24.00 |
| Yellow Beeswax | 6.00 |
| Vegetable Flakes | 6.00 |
| Glyceryl mono oleate | 2.70 |
| Span 60 | 0.12 |
| Lecithin | 0.30 |
| Methyl Cellulose | 3.00 |
| PEG 3350 | 9.00 |
| PEG 400 | 16.50 |

**Result:** T₅₀ (time required for 50% dissolution) is about 10h.

**Formulation 10:**

| *Ingredients* | *Amount (% w*/*w)* |
|---|---|
| Diltiazem Hydrochloride | 10.00 |
| Soybean Oil | 18.65 |
| Vegetable Shortening | 20.00 |
| Yellow Beeswax | 5.00 |
| Vegetable Flakes | 5.00 |
| Glyceryl mono oleate | 3.00 |
| Span 60 | 0.12 |
| Lecithin | 0.30 |
| Methyl Cellulose | 3.00 |
| PEG 3350 | 9.00 |
| PEG 400 | 16.50 |

**Result:** T₅₀ (time required for 50% dissolution) is about 3.5h.

**Formulation 11:**

| *Ingredients* | *Amount (% w*/*w)* |
|---|---|
| Diltiazem Hydrochloride | 10.00 |
| Soybean Oil | 23.38 |
| Vegetable Shortening | 24.00 |
| Yellow Beeswax | 6.00 |
| Vegetable Flakes | 6.00 |
| Glyceryl mono oleate | 2.70 |
| Span 60 | 0.12 |
| Lecithin | 0.30 |
| Methyl Cellulose | 3.00 |
| PEG 3350 | 15.00 |
| PEG 400 | 10.50 |

**Result:** T₅₀ (time required for 50% dissolution) is about >24h.

**Formulation 12:**

| *Ingredients* | *Amount (% w*/*w)* |
|---|---|
| Diltiazem Hydrochloride | 10.00 |
| Soybean Oil | 18.65 |
| Vegetable Shortening | 20.00 |
| Yellow Beeswax | 5.00 |
| Vegetable Flakes | 5.00 |
| Glyceryl mono oleate | 3.00 |
| Span 60 | 0.12 |
| Lecithin | 0.30 |
| Methyl Cellulose | 3.00 |
| PEG 3350 | 15.00 |
| PEG 400 | 10.50 |

**Result:** T₅₀ (time required for 50% dissolution) is about >24h.

**Formulation 13:**

| *Ingredients* | *Amount (% w*/*w)* |
|---|---|
| Diltiazem Hydrochloride | 10.00 |
| Soybean Oil | 10.39 |
| Vegetable Shortening | 31.99 |
| Yellow Beeswax | 8.00 |
| Vegetable Flakes | 8.00 |
| Glyceryl mono oleate | 2.70 |
| Span 60 | 0.12 |
| Lecithin | 0.30 |
| Methyl Cellulose | 3.00 |
| PEG 3350 | 9.00 |
| PEG 400 | 16.50 |

**Result:** T₅₀ (time required for 50% dissolution) is about 6.5h.

**Formulation 14:**

| *Ingredients* | *Amount (% w*/*w)* |
|---|---|
| Diltiazem Hydrochloride | 10.00 |
| Soybean Oil | 8.66 |
| Vegetable Shortening | 26.67 |
| Yellow Beeswax | 6.67 |
| Vegetable Flakes | 6.67 |
| Glyceryl mono oleate | 3.00 |
| Span 60 | 0.10 |
| Lecithin | 0.25 |
| Methyl Cellulose | 4.00 |
| PEG 3350 | 12.00 |
| PEG 400 | 22.00 |

**Result:** T₅₀ (time required for 50% dissolution) is about 3.5h.

**Formulation 15:**

| *Ingredients* | *Amount (% w*/*w)* |
|---|---|
| Diltiazem Hydrochloride | 10.00 |
| Soybean Oil | 10.34 |
| Vegetable Shortening | 32.00 |
| Yellow Beeswax | 8.00 |
| Vegetable Flakes | 8.00 |
| Glyceryl mono oleate | 2.50 |
| Span 60 | 0.10 |
| Lecithin | 0.30 |
| Methyl Cellulose | 3.00 |
| PEG 3350 | 15.00 |
| PEG 400 | 10.50 |

**Result:** T₅₀ (time required for 50% dissolution) is about >24h.

**Formulation 16:**

| *Ingredients* | *Amount (% w*/*w*) |
|---|---|
| Diltiazem Hydrochloride | 10.00 |
| Soybean Oil | 8.66 |
| Vegetable Shortening | 26.67 |
| Yellow Beeswax | 6.67 |
| Vegetable Flakes | 6.67 |
| Glyceryl mono oleate | 3.00 |
| Span 60 | 0.10 |
| Lecithin | 0.25 |
| Methyl Cellulose | 4.00 |
| PEG 3350 | 20.00 |
| PEG 400 | 14.00 |

**Result:** T₅₀ (time required for 50% dissolution) is about 6.5h.

**Formulation 17:**

| *Ingredients* | *Amount (% w*/*w)* |
|---|---|
| Diltiazem Hydrochloride | 10.00 |
| Soybean Oil | 46.34 |
| Vegetable Shortening | 8.00 |
| Yellow Beeswax | 2.00 |
| Vegetable Flakes | 2.00 |
| Glyceryl mono oleate | 2.70 |
| Span 60 | 0.12 |
| Lecithin | 0.30 |
| Methyl Cellulose | 3.00 |
| PEG 3350 | 15.00 |
| PEG 400 | 10.50 |

**Result:** T₅₀ (time required for 50% dissolution) is about 1.5h.

**Formulation 18:**

| *Ingredients* | *Amount (% w*/*w)* |
|---|---|
| Diltiazem Hydrochloride | 10.00 |
| Soybean Oil | 38.66 |
| Vegetable Shortening | 6.67 |
| Yellow Beeswax | 1.67 |
| Vegetable Flakes | 1.67 |
| Glyceryl mono oleate | 3.00 |
| Span 60 | 0.10 |
| Lecithin | 0.25 |
| Methyl Cellulose | 4.00 |
| PEG 3350 | 20.00 |
| PEG 400 | 14.00 |

**Result:** T₅₀ (time required for 50% dissolution) is about 1.5h.

**Formulation 19:**

| *Ingredients* | *Amount (% w*/*w)* |
|---|---|
| Diltiazem Hydrochloride | 10.00 |
| Soybean Oil | 34.34 |
| Vegetable Shortening | 16.00 |
| Yellow Beeswax | 4.00 |
| Vegetable Flakes | 4.00 |
| Glyceryl mono oleate | 2.70 |
| Span 60 | 0.12 |
| Lecithin | 0.30 |
| Methyl Cellulose | 3.00 |
| PEG 3350 | 15.00 |
| PEG 400 | 10.50 |

**Result:** T₅₀ (time required for 50% dissolution) is about 20h.

**Formulation 20:**

| *Ingredients* | *Amount (% w*/*w)* |
|---|---|
| Diltiazem Hydrochloride | 10.00 |
| Soybean Oil | 28.66 |
| Vegetable Shortening | 13.33 |
| Yellow Beeswax | 3.33 |
| Vegetable Flakes | 3.33 |
| Glyceryl mono oleate | 3.00 |
| Span 60 | 0.10 |
| Lecithin | 0.25 |
| Methyl Cellulose | 4.00 |
| PEG 3350 | 20.00 |
| PEG 400 | 14.00 |

**Result:** T₅₀ (time required for 50% dissolution) is about 20h.

**Formulation 21:**

| *Ingredients* | *Amount (% w*/*w)* |
|---|---|
| Diltiazem Hydrochloride | 5.00 |
| Soybean Oil | 12.46 |
| Vegetable Shortening | 52.50 |
| Vegetable Flakes | 3.50 |
| Glyceryl mono oleate | 2.65 |
| Span 60 | 0.20 |
| Methyl Cellulose | 2.50 |
| PEG 900 | 15.75 |
| PEG 400 | 5.25 |

**Result:** T₅₀ (time required for 50% dissolution) is about 0.3h.

**Formulation 22:**

| *Ingredients* | *Amount (% w*/*w)* |
|---|---|
| Diltiazem Hydrochloride | 5.00 |
| Soybean Oil | 9.79 |
| Vegetable Shortening | 27.50 |
| Vegetable Flakes | 2.75 |
| Glyceryl mono oleate | 2.75 |
| Glyceryl mono stearate | 2.00 |
| Span 60 | 1.00 |
| Methyl Cellulose | 4.00 |
| PEG 900 | 8.40 |
| PEG 400 | 25.20 |

**Result:** T₅₀ (time required for 50% dissolution) is about 0.3h.

**Reference Formulation 23:**

| *Ingredients* | *Amount (% w*/*w)* |
|---|---|
| Famotidine | 1.00 |
| Soybean Oil | 12.00 |
| Vegetable Shortening | 15.00 |
| Vegetable Flakes | 1.50 |
| Glyceryl mono oleate | 1.50 |
| Span 60 | 0.06 |
| Methyl Cellulose | 6.90 |
| Cremophor RH 40 | 0.69 |
| Glyceryl mono stearate | 3.45 |
| PEG 400 | 57.96 |

**Result:** T₅₀ (time required for 50% dissolution) is about 0.6h.

**Reference Formulation 24:**

| *Ingredients* | *Amount (% w*/*w)* |
|---|---|
| Vegetable Shortening | 25.00 |
| Methyl Cellulose | 11.30 |
| Cremophor RH 40 | 0.70 |
| Glyceryl mono stearate | 3.50 |
| PEG 400 | 59.50 |

**Formulation 25 (Dual Release):**

| *Ingredients* | *Amount* (% w/w) |
|---|---|
| Diltiazem Hydrochloride | 10.33 |
| Soybean Oil | 36.15 |
| Vegetable Shortening | 10.74 |
| Yellow Beeswax | 2.69 |
| Vegetable Flakes | 2.69 |
| Glyceryl mono oleate | 2.87 |
| Span 60 | 0.11 |
| Lecithin | 0.27 |
| Methyl Cellulose | 3.60 |
| PEG 3350 | 17.98 |
| PEG 400 | 12.59 |

### Procedure:

Vegetable shortening, vegetable flakes, yellow beeswax, glyceryl mono oleate, lecithin, Span 60 and soybean oil were melted together at 50° to 70°C (wax phase). Methylcellulose, PEG 3350 and PEG 400 were melted separately at 50° to 70°C (aqueous phase). About 77% of diltiazem hydrochloride was dispersed in the melted aqueous phase and added slowly to the wax phase with homogenization, while maintaining the temperature between 50° and 70°C. Remaining 23% of diltiazem hydrochloride was added to the final resultant homogeneous emulsion. The emulsion was cooled and encapsulated.

### Evaluation:

Filled capsules were subjected for dissolution as per USP using paddle method in distilled water at 100 RPM.

**Result:** T₅₀ (time required for 50% dissolution) is about 4.2h.

**Formulation 26:**

| *Ingredients* | *Amount (%* w/w) |
|---|---|
| Oxycodone Hydrochloride | 5.00 |
| Soybean Oil | 36.56 |
| Vegetable Shortening | 11.00 |
| Yellow Beeswax | 2.75 |
| Vegetable Flakes | 2.75 |
| Glyceryl mono oleate | 3.35 |
| Span 60 | 0.55 |
| Lecithin | 0.28 |
| Methyl Cellulose | 4.00 |
| PEG 3350 | 20.00 |
| PEG 400 | 14.00 |

**Procedure & Evaluation:** Procedure adopted was as described in Formulation 1.

**Result:** T₅₀ (time required for 50% dissolution) is about 3.5h.

**Reference Formulation 27:**

| *Ingredients* | *Amount (% w*/*w)* |
|---|---|
| Oxycodone Hydrochloride | 5.00 |
| Water | 6.00 |
| Soybean Oil | 36.56 |
| Vegetable Shortening | 11.00 |
| Yellow Beeswax | 2.75 |
| Vegetable Flakes | 2.75 |
| Glyceryl mono oleate | 3.10 |
| Span 60 | 0.55 |
| Lecithin | 0.28 |
| Methyl Cellulose | 4.00 |
| PEG 3350 | 20.00 |
| PEG 400 | 8.00 |

### Procedure:

Procedure adopted was similar to Formulation 25, but the model drug was dissolved in water before adding to the rest of the formulation.

### Evaluation:

Filled capsules were subjected for dissolution as per USP using paddle method in distilled water at 100 RPM.

**Result:** T₅₀ (time required for 50% dissolution) is about >8h.

**Reference Formulation 28:**

| *Ingredients* | *Amount (% w*/*w)* |
|---|---|
| Theophylline | 10.00 |
| Soybean Oil | 36.36 |
| Vegetable Shortening | 45.45 |
| Vegetable Flakes | 3.64 |
| Glyceryl Mono oleate | 4.54 |
| Cremophor EL 40 | 0.91 |

### Procedure:

Vegetable shortening, vegetable flakes, GMO, and Cremophor EL 40 were melted with soybean oil between 50 and 70°C. To this melted mass, theophylline was added and homogenized. The resultant mixture was cooled while mixing and encapsulated.

**Result:** T₅₀ (time required for 50% dissolution) is about 1h.

**Reference Formulation 29:**

| *Ingredients* | *Amount (% w*/*w)* |
|---|---|
| Theophylline | 10.00 |
| Soybean Oil | 36.36 |
| Vegetable Shortening | 45.45 |
| Vegetable Flakes | 4.32 |
| Glyceryl Mono oleate | 4.54 |
| Cremophor RH 40 | 0.23 |

**Procedure:** Procedure adopted was similar to Formulation 28.

**Result:** T₅₀ (time required for 50% dissolution) is about >24h.

**Reference Formulation 30:**

| *Ingredients* | *Amount (% w*/*w)* |
|---|---|
| Theophylline | 10.00 |
| Soybean Oil | 36.36 |
| Vegetable Shortening | 45.45 |
| Vegetable Flakes | 3.86 |
| Glyceryl Mono oleate | 4.54 |
| Cremophor RH 40 | 0.68 |

**Procedure:** Procedure adopted was similar to Formulation 28.

**Result:** T₅₀ (time required for 50% dissolution) is about 16h.

**Reference Formulation 31:**

| *Ingredients* | *Amount (% w*/*w)* |
|---|---|
| Theophylline | 10.00 |
| Soybean Oil | 36.36 |
| Vegetable Shortening | 45.45 |
| Vegetable Flakes | 4.09 |
| Glyceryl Mono oleate | 4.54 |
| Cremophor RH 40 | 0.45 |

**Procedure:** Procedure adopted was similar to Formulation 28.

**Result:** T₅₀ (time required for 50% dissolution) is about 12h.

## Claims

1. A controlled release soft capsule having a shell and a matrix fill comprising an active ingredient or drug, wherein the matrix fill consists of two phases in the form of an emulsion, wherein the emulsion comprises a hydrophilic internal phase consisting essentially of polyethylene glycol of molecular weight ranging from about 200 to about 8000, methyl cellulose and an active ingredient or drug and a lipid or lipophilic external phase; and
wherein the ratio of the internal phase to external phase is from about 0.5:10 to about 1:1 by weight.

2. The controlled release soft capsule of claim 1, wherein the external phase comprises a vegetable oil, hydrogenated vegetable oil, fatty acid, wax, fatty acid ester, or a combination thereof.

3. The controlled release soft capsule of claim 1 or 2, wherein the internal phase is structured.

4. The controlled release soft capsule of claim 3, wherein the internal phase is solid, semi-solid or gel.

5. The controlled release soft capsule of any one of the preceding claims, wherein the active ingredient or drug is dispersed in the internal phase as a solution or suspension form.

6. The controlled release soft capsule of any one of the preceding claims, wherein the ratio of internal phase to external phase is from 1:9 to 1:1 by weight.

7. The controlled release soft capsule of any one of the preceding claims, wherein the ratio of the active ingredient or drug to the matrix fill is from about 1:100 to about 1:2 by weight.

8. The controlled release soft capsule of any one of the preceding claims, wherein the emulsion comprises a surfactant or combination of surfactants having HLB values ranging from about 2 to about 20.

9. The controlled release soft capsule of any one of the preceding claims, wherein the active ingredient or drug is distributed in both an external and internal phase.

10. The controlled release soft capsule of claim 9, wherein the active ingredient or drug is in the form of solid particles.

11. The controlled release soft capsule of any one of the preceding claims, wherein the active ingredient or drug is selected from the group consisting of anti-asthmatics, narcotic analgesics, narcotic antagonists, and cardiovascular drugs and/or is selected from the group consisting of diltiazem, nifedipine, oxycodone, morphine, morphine analogues, and morphine antagonists.

12. A method of manufacturing a controlled release soft capsule according to claim 1, the method comprising
a) dispersing the active ingredient or drug in an internal phase to form a clear solution or suspension using a propeller or homogenizer mixer;
b) adding the internal phase to a molten external phase containing at least one surfactant in an amount from about 0.1% to about 5% by weight to form a resulting mixture;
c) forming an emulsion from the resulting mixture by subjecting the mixture to mechanical forces generated by a propeller mixer, a homogenizer, or a microfluidizer;
d) cooling the emulsion to from about 20°C to about 35°C; and
e) encapsulating the emulsion using a rotary die encapsulation machine to form the controlled release capsule.

## Patentansprüche

1. Eine Weichkapsel mit kontrollierter Freisetzung mit einer Hülle und einer Matrixfüllung, einen Wirkstoff oder ein Arzneimittel umfassend, wobei die Matrixfüllung aus zwei Phasen in der Form einer Emulsion besteht, wobei die Emulsion Folgendes umfasst:
eine hydrophile interne Phase, die im Wesentlichen aus Polyethylenglycol mit einem Molekülgewicht von etwa 200 bis etwa 8000, Methylcellulose und einem Wirkstoff oder einem Arzneimittel besteht, und eine Lipid- oder lipophile externe Phase; und
wobei das Verhältnis von der internen Phase zur externen Phase von etwa 0,5:10 bis etwa 1:1 nach Gewicht beträgt.

2. Weichkapsel mit kontrollierter Freisetzung nach Anspruch 1, wobei die externe Phase ein Pflanzenöl, ein gehärtetes Pflanzenöl, eine Fettsäure, ein Wachs, einen Fettsäureester oder eine Kombination aus diesen umfasst.

3. Weichkapsel mit kontrollierter Freisetzung nach Anspruch 1 oder 2, wobei die interne Phase strukturiert ist.

4. Weichkapsel mit kontrollierter Freisetzung nach Anspruch 3, wobei die interne Phase fest, halbfest oder gelförmig ist.

5. Weichkapsel mit kontrollierter Freisetzung nach einem der vorhergehenden Ansprüche, wobei der Wirkstoff oder das Arzneimittel als eine Lösungs- oder Suspensionsform in der internen Phase dispergiert ist.

6. Weichkapsel mit kontrollierter Freisetzung nach einem der vorhergehenden Ansprüche, wobei das Verhältnis von interner Phase zu externer Phase von 1:9 bis 1:1 nach Gewicht beträgt.

7. Weichkapsel mit kontrollierter Freisetzung nach einem der vorhergehenden Ansprüche, wobei das Verhältnis zwischen Wirkstoff oder Arzneimittel und Matrixfüllung von etwa 1:100 bis etwa 1:2 nach Gewicht beträgt.

8. Weichkapsel mit kontrollierter Freisetzung nach einem der vorhergehenden Ansprüche, wobei die Emulsion ein Tensid oder eine Kombination aus Tensiden mit von etwa 2 bis etwa 20 reichenden HLB-Werten umfasst.

9. Weichkapsel mit kontrollierter Freisetzung nach einem der vorhergehenden Ansprüche, wobei der Wirkstoff oder das Arzneimittel sowohl in einer externen als auch in einer internen Phase verteilt ist.

10. Weichkapsel mit kontrollierter Freisetzung nach Anspruch 9, wobei der Wirkstoff oder das Arzneimittel in Form von Feststoffpartikeln vorliegt.

11. Weichkapsel mit kontrollierter Freisetzung nach einem der vorhergehenden Ansprüche, wobei der Wirkstoff oder das Arzneimittel ausgewählt ist aus der Gruppe bestehend aus Antiasthmatika, narkotischen Schmerzmitteln, narkotischen Antagonisten und kardiovaskulären Arzneimitteln und/oder ausgewählt ist aus der Gruppe bestehend aus Diltiazem, Nifedipin, Oxycodon, Morphin, Morphinanaloga und Morphinantagonisten.

12. Verfahren zum Herstellen einer Weichkapsel mit kontrollierter Freisetzung nach Anspruch 1, wobei das Verfahren Folgendes umfasst:
a) Dispergieren des Wirkstoffs oder des Arzneimittels in einer internen Phase, um unter Einsatz eines Propeller- oder Homogenisatormischers eine klare Lösung oder Suspension auszubilden;
b) Hinzufügen der internen Phase zu einer geschmolzenen externen Phase, wenigstens ein Tensid in einer Menge von etwa 0,1 Gew.-% bis etwa 5 Gew.-% enthaltend, um eine sich ergebende Mischung auszubilden;
c) Ausbilden einer Emulsion aus der sich ergebenden Mischung durch Aussetzen der Mischung durch einen Propellermischer, einen Homogenisator oder einen Microfluidizer erzeugten mechanischen Kräften;
d) Abkühlen der Emulsion auf von etwa 20 °C bis etwa 35 °C; und
e) Einkapseln der Emulsion unter Einsatz einer Rotationsstanzen-Einkapselungsmaschine, um die Kapsel mit kontrollierter Freisetzung auszubilden.

## Revendications

1. Capsule molle à libération contrôlée ayant une enveloppe et une charge matricielle comprenant un principe actif ou un médicament, dans laquelle la charge matricielle est constituée de deux phases sous la forme d'une émulsion, dans laquelle l'émulsion comprend une phase interne hydrophile constituée principalement de polyéthylène glycol d'un poids moléculaire compris entre environ 200 et environ 8 000, de méthylcellulose et d'un principe actif ou d'un médicament, et d'une phase externe lipidique ou lipophile ; et dans laquelle le rapport de la phase interne à la phase externe est compris entre environ 0,5:10 et environ 1:1 en poids.

2. Capsule molle à libération contrôlée selon la revendication 1, dans laquelle la phase externe comprend une huile végétale, une huile végétale hydrogénée, un acide gras, une cire, un ester d'acide gras ou une combinaison de ceux-ci.

3. Capsule molle à libération contrôlée selon la revendication 1 ou 2, dans laquelle la phase interne est structurée.

4. Capsule molle à libération contrôlée selon la revendication 3, dans laquelle la phase interne est solide, semi-solide ou gélifiée.

5. Capsule molle à libération contrôlée selon l'une quelconque des revendications précédentes, dans laquelle le principe actif ou le médicament est dispersé dans la phase interne sous la forme d'une solution ou d'une suspension.

6. Capsule molle à libération contrôlée selon l'une quelconque des revendications précédentes, dans laquelle le rapport de la phase interne à la phase externe est compris entre 1:9 et 1:1 en poids.

7. Capsule molle à libération contrôlée selon l'une quelconque des revendications précédentes, dans laquelle le rapport du principe actif ou du médicament à la charge matricielle est compris entre environ 1:100 et environ 1:2 en poids.

8. Capsule molle à libération contrôlée selon l'une quelconque des revendications précédentes, dans laquelle l'émulsion comprend un tensioactif ou une combinaison de tensioactifs ayant des indices HLB compris entre environ 2 et environ 20.

9. Capsule molle à libération contrôlée selon l'une quelconque des revendications précédentes, dans laquelle le principe actif ou le médicament est réparti à la fois dans une phase externe et dans une phase interne.

10. Capsule molle à libération contrôlée selon la revendication 9, dans laquelle le principe actif ou le médicament est sous la forme de particules solides.

11. Capsule molle à libération contrôlée selon l'une quelconque des revendications précédentes, dans laquelle le principe actif ou le médicament est choisi dans le groupe constitué par les antiasthmatiques, les analgésiques narcotiques, les antagonistes narcotiques et les médicaments cardiovasculaires et/ou est choisi dans le groupe constitué par le diltiazem, la nifédipine, l'oxycodone, la morphine, les analogues de la morphine et les antagonistes de la morphine.

12. Procédé de fabrication d'une capsule molle à libération contrôlée selon la revendication 1, le procédé comprenant les étapes consistant à :
a) disperser le principe actif ou le médicament dans une phase interne pour former une solution ou une suspension limpide à l'aide d'un agitateur à hélices ou d'un homogénéisateur ;
b) ajouter la phase interne à une phase externe fondue contenant au moins un tensioactif dans une quantité comprise entre environ 0,1 % et environ 5 % en poids pour former un mélange résultant ;
c) former une émulsion à partir du mélange résultant en soumettant le mélange à des forces mécaniques générées par un agitateur à hélices, un homogénéisateur ou un microfluidiseur ;
d) refroidir l'émulsion entre environ 20 °C et environ 35 °C ; et
e) encapsuler l'émulsion à l'aide d'une machine d'encapsulation à matrice rotative pour former la capsule à libération contrôlée.
